# EUROPÄISCHE PATENTSCHRIFT

(11) **EP 0 648 733 B1**
(45) Veröffentlichungstag und Bekanntmachung des Hinweises auf die Patenterteilung: **21.01.1998**
(21) Anmeldenummer: 94115808.1
(22) Anmeldetag: 07.10.1994
(51) Int. Cl.: C07C 67/02, C07C 227/18, C07D 471/04

(54) **Verfahren zur Herstellung von Zwischenprodukten der Chinacridonsynthese**
Process for the preparation of intermediates in the quinacridone synthesis
Procédé pour la préparation des produits intermédiaires dans la synthèse de quinacridone

(30) Priorität: 19.10.1993 AT 2096/93
(43) Veröffentlichungstag der Anmeldung: 19.04.1995
(73) Patentinhaber: DSM Chemie Linz GmbH, 4021 Linz (AT)
(72) Erfinder: Schwarz, Franz Thomas, Dipl.-Ing., A-4493 Wolfern (IT); Altreiter, Johann, A-4212 Neumarkt (IT); Möstl, Franz, A-4173 St. Veit (IT)
(74) Vertreter: Kunz, Ekkehard, Dr.

(56) Entgegenhaltungen:
- EP-A- 0 536 083
- WO-A-92/09558
- GB-A- 891 640
- GB-A- 1 228 727
- US-A- 2 821 541
- US-A- 3 674 814
- US-A- 4 124 768
- US-A- 4 435 589
- US-A- 4 956 464
- US-A- 4 981 997
- HELVETICA CHIMICA ACTA, Bd.62, Nr.5, 1979 Seiten 1682 - 1687 J. SINNREICH ET AL. 'Chemistry of Succinyl Succinic Acid Derivatives. Part V. On the Transesterification of Succinyl Succinates'

## Beschreibung

Chinacridone haben vornehmlich als Rot- oder Violettpigmente in der Farbstoffindustrie große Bedeutung erlangt.
In GB-PS 891,640 ist das grundlegende Reaktionsschema geoffenbart, nach dem Chinacridone im allgemeinen hergestellt werden:
1. Kondensation von Diethylsuccinat zum entsprechenden Diethylsuccinylsuccinat, das
2. mit einem Anilin zu 2,5-Dianilino-3,6-dihydroterephthalsäurediethylester umgesetzt wird. Dieses wird
3. zum 2,5-Dianilinoterephthalsäurediethylester oxidiert, der
4. zur 2,5-Dianilinoterephthalsäure verseift wird. Diese wird schließlich
5. zum Chinacridon ringgeschlossen.

Dem angegebenen Reaktionsschema folgen eine Reihe von Verfahren, die bezüglich einzelner oder mehrerer Reaktionsstufen Verbesserungen gegenüber dem Verfahren der GB-PS 891,640 erbringen sollen. In den bekannten Verfahren werden aber Lösungsmittel oder Reagentien verwendet, deren Anwendung vom Standpunkt des Umweltschutzes bedenklich erscheint.

Gemäß GB-PS 891,640 selbst wird die Oxidation und Verseifung in den Reaktionsstufen 3. und 4. in Pyridin, Picolinen oder deren Homologen vorgenommen, deren Anwendung und Entsorgung aber unangenehm und nicht umweltschonend sind. Gemäß den in US-PS 2.821.541 oder US-PS 3,674,814 beschriebenen Verfahrensweisen wird "Dowtherm A", eine Mischung aus Biphenyl und Diphenyloxid, gemäß WO 92/09558 wird Xylol als Lösungsmittel eingesetzt. Solche Lösungsmittel haben aber toxische Eigenschaften. In US-PS 3,031,501, US-PS 3,388,149, GB-PS 1,228,727 oder US-PS 4,124,768 ist die Oxidation gemäß der Reaktionsstufe 3. mit Hilfe von hochgiftigen Nitrobenzolen beschrieben.

In US-PS 4,981,997 ist zur Oxidation in Reaktionsstufe 3 die Verwendung eines sauerstoffübertragendes Mittels wie Anthrachinon-2-sulfonsäure und einer quaternären Ammoniumverbindung beschrieben. Solche Verbindungen sind aber nur schwer, wenn überhaupt, aus dem Reaktionsprodukt zu entfernen.

In US-PS 4,435,589 wird ein Verfahren gemäß dem beschriebenen Reaktionsschema, ausgehend von Dimethylsuccinat in Methanol als Lösungsmittel beschrieben, bei dem die Oxidation gemäß Stufe 3 mit Hilfe von Luft erfolgt. Die Ausbeuten der dabei hergestellten 2,5-Dianilinoterephthalsäuren betragen aber nur etwa 70 %. Die geringen Ausbeuten sind gemäß EP-A1-0 536 083 auf die geringe Löslichkeit von Dimethylsuccinat und dessen Folgeprodukten, die gemäß dem beschriebenen Reaktionsschema gebildet werden, in einem Lösungsmittel zurückzuführen. In EP-A1-0 536 083 wird daher vorgeschlagen, Dimethylsuccinat mit Hilfe von aliphatischen Alkoholen, die 2 bis 6 C-Atome aufweisen, umzuestern und die Umesterungsprodukte, die infolge der längeren hydrophoben Seitenketten eine höhere Löslichkeit in organischen Lösungsmitteln aufweisen, zur Herstellung von Chinacridonen gemäß dem beschriebenen Reaktionsschema einzusetzen. Gemäß der Beispiele gelingt jedoch die Umsetzung, die in Gegenwart von Metallalkoholat durchgeführt wird, in keinem Fall vollständig, sodaß das Reaktionsgemisch stets ansehnliche Mengen von unumgesetztem Dimethylsuccinat enthält. Die Löslichkeit des dementsprechend hergestellten Dialkyl-, Methylalkyl- und Dimethylsuccinatgemisches scheint in organischen Lösungsmitteln gegenüber jener von Dimethylsuccinat allein nicht sehr verbessert, denn gemäß der Beispiele muß auch zur Umsetzung der umgeesterten Succinate die bereits beschriebene toxische Mischung aus Biphenyl und Diphenyloxid (Dowtherm A) verwendet werden.

Es wurde nun unerwarteterweise gefunden, daß Dimethylsuccinylsuccinat mit einem Alkohol, der mindestens 2 C-Atome aufweist, umgeestert werden kann, wobei praktisch kein Dimethylsuccinylsuccinat unreagiert zurückbleibt. Dies war gänzlich unerwartet, denn aus Helvetica Chimica Acta, Vol. 62, Fasc. 5 (1979), pp. 1682 bis 1687 ist bekannt, daß Dimethylsuccinylsuccinat im Gegensatz zu Diethylsuccinylsuccinat infolge der außergewöhnlichen Stabilität der Methylesterbindungen nicht umgeestert werden kann.
Die erfindungsgemäß umgeesterten Succinylsuccinate und ihre Folgeprodukte gemäß obigem Reaktionsschema weisen dabei eine bessere Löslichkeit als Dimethylsuccinylsuccinat und dessen Folgeprodukte in umweltschonenden organischen Lösungsmitteln, wie Alkoholen auf und können daher in ein umweltschonenden Verfahren zur Herstellung von 2,5-Dianilinoterephthalsäure in einem alkoholischen Lösungsmittel eingesetzt werden, wobei die Ausbeuten unerwartet hoch sind. Die auf diese Weise gemäß dem beschriebenen Reaktionsschema in umweltschonenden Lösungsmitteln hergestellten 2,5-Dianilinoterephthalsäuren werden wie üblich zu den Chinacridonen ringgeschlossen.
Gegenstand der Erfindung ist daher ein Verfahren zur Umesterung von Dimethylsuccinylsuccinat, das dadurch gekennzeichnet ist, daß Dimethylsuccinylsuccinat mit einem oder mehreren unsubstituierten oder durch Phenylgruppen substituierten aliphatischen, geradkettigen, verzweigten oder cyclischen Monoalkoholen, die jeweils 2 bis 22 C-Atome aufweisen, in Gegenwart eines sauren Katalysators mit oder ohne die Anwesenheit eines unter den Reaktionsbedingungen inerten Verdünnungsmittels unter Sauerstoffausschluß und unter Druck umgesetzt wird, wobei zumindest eine der beiden Methoxygruppen durch die dem verwendeten Alkohol entsprechende Oxygruppe ersetzt wird.

Dimethylsuccinylsuccinat kann durch Kondensation von 2 Mol Dimethylsuccinat, das ein leich erhältliches, großtechnisch erzeugtes Produkt darstellt, wie üblich hergestellt werden.

Unter Alkohol ist ein geradkettiger, verzweigter oder cyclischer aliphatischer Monoalkohol, der 2 bis 22, bevorzugt 2 bis 16, besonders bevorzugt 3 bis 8 C-Atome aufweist, beispielsweise Ethanol, Propanol, Butanol, Hexanol, Octanol, Dodecanol, Eicosanol oder ihre Isomeren wie iso-Propanol, iso-Butanol, 2-Methylhexanol, Cyclopentanol, Cyclohexanol, Cyclooctanol oder eine Mischung solcher Alkohole zu verstehen, wobei der Alkohol unsubstituiert oder durch Arylgruppen, bevorzugt Phenylgruppen, wie beispielsweise Benzylalkohol substituiert sein kann. Bevorzugt wird ein unsubstituierter, aliphatischer, geradkettiger oder verzweigter Alkohol mit 3 bis 8 C-Atomen eingesetzt. Bevorzugt wird nur ein Alkohol eingesetzt und nicht mehrere Alkohole.

Als saurer Katalysator werden Mineralsäuren, beispielsweise Schwefelsäure, Salzsäure, Salpetersäure, bevorzugt Schwefelsäure, stark saure Kationenaustauscher oder organische Sulfonsäuren, wie p-Toluolsulfonsäure, bevorzugt Mineralsäuren eingesetzt.

Die Umsetzung kann direkt im gewünschten Alkohol stattfinden, sofern dieser unter den Reaktionsbedingungen flüssig ist, oder es wird ein unter den Reaktionsbedingungen inertes Verdünnungsmittel zugesetzt. Als inerte Verdünnungsmittel kommen beispielsweise aliphatische Kohlenwasserstoffe, wie Pentan, Hexan, Cyclopentan, Cyclohexan, Ether, wie Tetrahydrofuran oder Mischungen solcher Verdünnungsmittel in Betracht.

Zur Durchführung der erfindungsgemäßen Reaktion wird Dimethylsuccinylsuccinat mit dem gewünschten Alkohol, dem sauren Katalysator und, falls erwünscht, dem Verdünnungsmittel gemischt und unter Sauerstoffausschluß unter Druck erhitzt.

Pro Mol Dimethylsuccinylsuccinat werden mindestens 2 Mol Alkohol, im allgemeinen aber ein Überschuß von mindestens 5 Mol Alkohol pro Mol Dimethylsuccinylsuccinat eingesetzt. Ist der Alkohol unter den Reaktionsbedingungen flüssig, kann die Reaktion direkt im gewünschten Alkohol durchgeführt werden, wobei in diesem Fall mindestens die vierfache molare Menge Alkohol bezogen auf Dimethylsuccinylsuccinat eingesetzt wird.
Die optimale Menge des Verdünnungsmittels ist durch einfache Vorversuche mit verschiedenen Mengen leicht zu ermitteln. Der saure Katalysator wird in katalystischen Mengen, d.h. in Mengen von etwa 0,1 bis 10 Molprozent bezogen auf Dimethylsuccinylsuccinat eingesetzt.

Die Reaktionsmischung wird in einem drucksicheren Gefäß unter Sauerstoffausschluß erhitzt. Dazu wird eine Inertgasatomosphäre beispeilsweise mit Hilfe von Stickstoff, Helium, Argon, wie üblich aufgebaut. Die Reaktionstemperaturen betragen von etwa 60 bis 220 °C, bevorzugt von 80 bis 210 °C, besonders bevorzugt von 90 bis 160 °C. Die Reaktion wird dabei unter dem entstehenden Druck ausgeführt, wobei eine Regelung mit Hilfe des Inertgasdruckes möglich ist. Der Druck beträgt im allgemeinen von etwa 1 bis 20 bar, bevorzugt 2 bis 6 bar.

Bei Durchführung des erfindungsgemäßen Verfahrens wird zumindest eine der beiden Methoxygruppen des Dimethylsuccinylsuccinat durch die entsprechende Alkoxygruppe des verwendeten Alkohols ersetzt, wobei praktisch kein unreagiertes Ausgangsprodukt zurückbleibt. Im allgemeinen entsteht ein Gemisch aus Dialkyl- und Alkylmethylsuccinylsuccinat, wobei der Alkylteil dem Alkylteil des zur Umsetzung eingesetzten Alkohols entspricht. Der Dialkylsuccinylsuccinatanteil im Produktgemisch wird dabei umso höher, je höher die Reaktionstemperatur und je länger die Reaktionszeit angesetzt wird. Wird ein Alkoholgemisch verwendet, entstehen Mischungen den verwendeten Alkoholen entsprechender Succinylsuccinate.

Der Reaktionsverlauf wird wie üblich, vorteilhaft chromatographisch verfolgt. Je nach gewünschtem Dialkylsuccinylsuccinatanteil im Produktgemisch, frühestens jedenfalls nach Verbrauch des Dimethylsuccinylsuccinats in der Reaktionsmischung, wird die Reaktion abgebrochen und die Reaktionsmischung abgekühlt. Dabei fallen die Reaktionsprodukte im allgemeinen kristallin aus. Sie werden wie üblich, etwa durch Filtration, Abzentrifugieren isoliert. In Spezialfällen kann eine Reinigung, etwa durch Umkristallisieren angeschlossen werden.

Das erfindungsgemäße Verfahren liefert Methylalkyl- und Dialkylsuccinylsuccinate in denen die Alkylgruppen jenen der zur Umsetzung verwendeten Alkohole entsprechen. Solche Succinylsuccinate sind in verschiedensten chemischen Synthesen einsetzbar. Vornehmlich werden die Produkte aber zur umweltschonenden Synthese von 2,5-Dianilinoterephthalsäuren, die eine Vorstufe bei der Chinacridonsynthese darstellen, eingesetzt. Bei dieser Synthese spielt es keine Rolle, daß gemäß dem erfindungsgemäßen Verfahren im allgemeinen Mischungen aus Methylalkyl- und Dialkylsuccinylsuccinaten entstehen, da die Ester auf der späteren Synthesestufe 4. des obigen Reaktionsschemas zur Säure verseift werden, sodaß jedenfalls ab der Verseifungsstufe einheitliche Produkte entstehen.

Gegenstand der Erfindung ist daher auch ein Verfahren zur Herstellung von 2,5-Dianilionterephthalsäuren der Formel 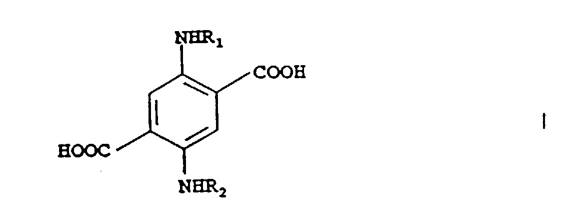 in der R₁ und R₂ gleich oder verschieden sind und jeweils eine unsubstituierte oder ein- oder mehrfach durch Halogen, Nitro-, Alkyl-, Alkoxy-, Phenyl-, Phenoxy-, Trifluormethyl oder durch alkylsubstituierte Carbamoylgruppen substituierte Phenyl- oder Naphthylgruppe bedeuten, wobei zumindest eine der alpha Positionen des C-Atoms, an das die Aminogruppe gebunden ist, unsubstituiert ist, das dadurch gekennzeichnet ist, daß
a) Dimethylsuccinylsuccinat mit einem oder mehreren Alkoholen der Formel

   R₃ - OH II

   in der R₃ eine unsubstituierte oder durch Phenyl substituierte Alkylgruppe mit 3 bis 22 C-Atomen bedeutet in Gegenwart eines sauren Katalysators unter Druck bei Temperaturen von 60 bis 180 °C erhitzt wird, wobei eine Verbindung der Formel 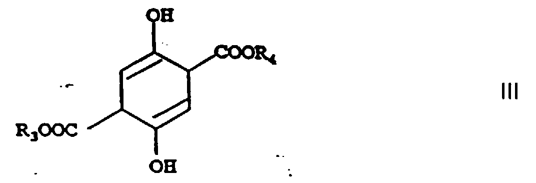 in der R₃ die obgenannte Bedeutung hat und R₄ die Bedeutung von R₃ hat und zusätzlich eine Methylgruppe bedeutet, wobei R₃ und R₄ in der Formel III austauschbar sind, erhalten wird,
b) das Reaktionsprodukt aus Stufe a) nach oder ohne Isolierung gegebenenfalls unter Zugabe eines unter den Reaktionsbedingungen inerten Verdünnungsmittels mit mindestens der zweifachen molaren Menge eines oder mehrerer Amine der Formel

   R₁-NH₂ und/oder R₂-NH₂ IV

   in der R₁ und R₂ die obgenannte Bedeutung haben in Gegenwart von Säure auf Temperaturen von 40 bis 150 °C erhitzt wird, wobei eine Verbindung der Formel 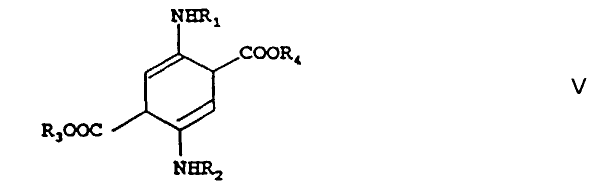 in der R₁, R₂, R₃ und R₄ die obgenannten Bedeutungen haben, erhalten wird,
c) das Reaktionsprodukt aus Stufe b) nach oder ohne Isolierung gegebenenfalls unter Zugabe eines unter den Reaktionsbedingungen inerten Verdünnungsmittels mit Säure bis zur sauren Reaktion versetzt und auf Temperaturen von 50 bis 100 °C erhitzt wird, worauf Luft in das Reaktionsgemisch eingeblasen wird, wobei der Terephthalsäureester der Formel 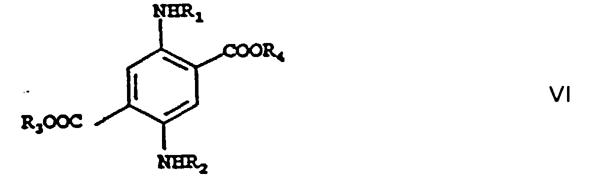 in der R₁, R₂, R₃ und R₄ die obgenannte Bedeutung haben, erhalten wird, der
d) nach oder ohne Isolierung gegebenenfalls unter Zugabe eines unter den Reaktionsbedingungen inerten Verdünnungsmittels durch Umsetzung mit einer Verbindung der Formel M-OH, in der M ein Metall bedeutet, unter Erhitzen in eine Verbindung der Formel 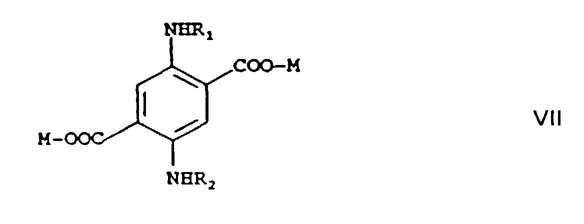 übergeführt wird, die
e) nach oder ohne Isolierung gegebenenfalls unter Zugabe eines unter den Reaktionsbedingungen inerten Verdünnungsmittels mit Wasser und Säure bis zur sauren Reaktion versetzt und zur Terephthalsäure der Formel I reagieren gelassen wird, die aus dem Reaktionsgemisch isoliert wird.

Das Verfahren mit Ausnahme der Stufe a. wird nach einer in einer der zitierten Literaturstellen geoffenbarten Art und Weise, aber in einem Alkohol der Formel II als Verdünnungsmittel oder in einem der für die Stufe a. angegebenen, inerten Verdünnungsmittel ausgeführt.
Die Reaktionsstufe a) wird erfindungsgemäß, wobei der Alkohol der Formel R₃OH, in der R₃ die obgenannte Bedeutung hat, bevorzugt gleichzeitig als Verdünnungsmittel eingesetzt wird, ausgeführt. Bevorzugt bedeutet R₃ eine geradkettige oder verzweigte Alkylgruppe mit 3 bis 8 C-Atomen. Bevorzugt wird nur ein Alkohol eingesetzt und nicht mehrere Alkohole.
Reaktionsstufe b) wird bevorzugt gemäß US-PS 4,435,589, aber unter Verwendung einer Verbindung der Formel III als Ausgangsprodukt und im Alkohol der Formel II oder im inerten Verdünnungsmittel der Stufe a., bevorzugt im Alkohol der Formel II als Verdünnungsmittel, die Reaktionsstufen c) und d) bevorzugt gemäß WO 92/09558, aber unter Verwendung einer Verbindung der Formel III bzw. der Formel V und ohne Verwendung eines Molybdän-Katalysators, ohne Isolierung des Terephthalsäureesters und im Alkohol der Formel II oder im inerten Verdünnungsmittel der Stufe a., bevorzugt im Alkohol der Formel II als Verdünnungsmittel durchgeführt. Bevorzugt wird nur ein Amin der Formel IV eingesetzt, wobei R₁ bevorzugt eine unsubstituierte oder durch Alkyl- oder Alkoxygruppen mit jeweils 1 bis 4 C-Atomen, Halogen oder Nitrogruppen substituierte Phenylgruppe bedeutet, wobei zumindest eine der alpha Positionen des C-Atoms, an das die Aminogruppe gebunden ist, unsubstituiert ist.
Die Reaktionsstufe e) wird bevorzugt durch Zugabe von Wasser und einer Mineralsäure zum Reaktionsgemisch bestehend aus dem Terephthalat der Formel VII im Alkohol der Formel II oder im inerten Verdünnungsmittel der Stufe a., bevorzugt im Alkohol der Formel II als Verdünnungsmittel ausgeführt.
Die Reaktionsschritte a. bis d. können dabei mit oder ohne Isolierung der einzelnen Reaktionsprodukte durchgeführt werden, wobei sich gezeigt hat, daß in einem Eintopfverfahren ohne Isolierung einzelner Reaktionsprodukte dennoch hohe Ausbeuten an Terephthalsäure der Formel VII von etwa 90 % bezogen auf Dimethylsuccinylsuccinat erzielt werden.

In einer besonders bevorzugten Ausführungsform wird Dimethylsuccinylsuccinat mit einem unsubstituierten, aliphatischen, geradkettigen oder verzweigten Alkohol, der 3 - 8 C-Atome aufweist und mit einer Mineralsäure in einem drucksicheren Gefäß in einer Inertgasatmosphäre auf Temperaturen von 90 bis 160°C erhitzt. Nach beendeter Reaktion wird der Reaktor entspannt und das Reaktionsgemisch mit einem Amin der Formel R₁-NH₂, in der R₁ eine durch eine oder mehrere Alkylgruppen substituierte Phenylgruppe bedeutet, wobei zumindest eine der alpha Positionen des C-Atoms, an das die Aminogruppe gebunden ist, unsubstituiert ist, auf Temperaturen von 60 bis 100°C erhitzt, worauf das Reaktionsgemisch angesäurert und auf Temperaturen von 60 bis 90 °C erhitzt wird. Anschließend wird in das Reaktionsgemisch Luft eingeblasen, bis die Reaktion zum Terephthalsäureester der Formel VI abgeschlossen ist. Danach wird der Reaktionsmischung ein Alkalimetallhydroxid bis zur alkalischen Reaktion zugegeben und auf Rückfluß erhitzt. Nach Abkühlen wird der Reaktionsmischung Wasser und eine Mineralsäure bis zur sauren Reaktion zugesetzt. Bilden sich dabei infolge der schlechten Mischbarkeit des verwendeten Verdünnungsmittels mit Wasser 2 Phasen aus, wird die wäßrige Phase abgetrennt, worauf die Terephthalsäure der Formel I aus der Reaktionsmischung im allgemeinen kristallin ausfällt und durch Filtration isoliert wird.

Da beim beschriebenen Verfahren keine umweltschädigenden Stoffe entsorgt werden müssen, stellt das Verfahren zur Herstellung von Dialkyl- und Alkylmethylsuccinylsuccinaten, von Terephthalsäuren der Formel eine Bereicherung der Technik dar.

### Beispiel

Ein 0,5 l Autoklav mit Rührer und Gaseinleitrohr wurde mit 46 g Dimethylsuccinylsuccinat (0,2 Mol), 250 ml 1-Butanol und 0,52 ml konzentrierter Schwefelsäure befüllt und verschlossen. Das Reaktionsgemisch wurde 15 Minuten mit Stickstoff gespült und 2 Stunden auf 140 °C erhitzt. Nach Abkühlen fiel ein Niederschlag aus, der dünnschichtchromatographisch als ein Gemisch aus Dibutylsuccinylsuccinat und Methyl-Butylsuccinylsuccinat identifiziert wurde. Dimethylsuccinylsuccinat wurde nur mehr in Spuren gefunden. Das Reaktionsgemisch wurde mit 26 g p-Toluidin versetzt, mit Stickstoff gespült und ohne Druck unter weiterer Stickstoffspülung 1 Stunde auf etwa 75 °C erwärmt. Danach wurden 20 ml Essigsäure zugegeben und auf Rückfluß erhitzt. Nach Erreichen der Rückflußtemperatur wurde mit Hilfe eines Tauchrohres 4 Stunden lang Luft in das Reaktionsgemisch eingeblasen. Anschließend wurden 70 g festes Kaliumhydroxid zugegeben und noch 1 Stunde auf Rückfluß erhitzt. Nach Abkühlen wurde der Reaktionsmischung 500 ml Wasser zugesetzt und der pH-Wert durch Zufügen von Schwefelsäure auf 2,5 bis 3 gestellt. Nach Abtrennen der wäßrigen Phase und erneuter Zugabe von etwa 400 ml Wasser fiel ein dunkelvioletter Niederschlag aus, der abfiltiert und bei etwa 100 °C getrocknet wurde.

Dabei wurden 67,6 g reine 2,3-Dimethyldianilino-terephthalsäure, das sind 90 % der Theorie bezogen auf Dimethylsuccinylsuccinat, erhalten. Die Reinheit wurde mittels HPLC (High pressure liquid chromatography) bestimmt und betrug 98 %.

## Patentansprüche

1. Verfahren zur Umesterung von Dimethylsuccinylsuccinat, dadurch gekennzeichnet, daß Dimethylsuccinylsuccinat mit einem oder mehreren unsubstituierten oder durch Phenylgruppen substituierten aliphatischen, geradkettigen, verzweigten oder cyclischen Monoalkoholen, die jeweils 2 bis 22 C-Atome aufweisen, in Gegenwart eines sauren Katalysators mit oder ohne die Anwesenheit eines unter den Reaktionsbedingungen inerten Verdünnungsmittels unter Sauerstoffausschluß und unter Druck umgesetzt wird, wobei zumindest eine der beiden Methoxygruppen durch die dem verwendeten Alkohol entsprechende Oxygruppe ersetzt wird.

2. Verfahren nach Anspruch 1, dadurch gekennzeichnet, daß als Alkohol ein unsubstituierter, aliphatischer, geradkettiger oder verzweigter Monoalkohol mit 2 bis 16 C-Atomen eingesetzt wird.

3. Verfahren nach Anspruch 2, dadurch gekennzeichnet, daß als Alkohol ein unsubstituierter, aliphatischer, geradkettiger oder verzweigter Monoalkohol mit 3 bis 8 C-Atomen eingesetzt wird.

4. Verfahren nach einem der Ansprüche 1 bis 3, dadurch gekennzeichnet, daß der Alkohol gleichzeitig als Verdünnungsmittel eingesetzt wird.

5. Verfahren nach einem der Ansprüche 1 bis 4, dadurch gekennzeichnet, daß als saurer Katalysator eine Mineralsäure oder ein stark saurer Kationenaustauscher eingesetzt wird.

6. Verfahren zur Herstellung von 2,5-Dianilinoterephthalsäuren der Formel 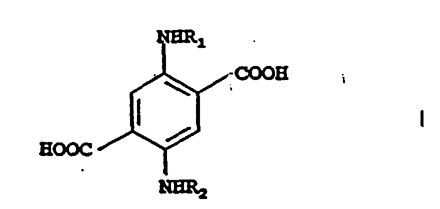 in der R₁ und R₂ gleich oder verschieden sind und jeweils eine unsubstituierte oder ein- oder mehrfach durch Halogen, Nitro-, Alkyl-, Alkoxy-, Phenyl-, Phenoxy-, Trifluormethyl oder durch alkylsubstituierte Carbamoylgruppen substituierte Phenyl- oder Naphthylgruppe bedeutet, wobei zumindest eine der alpha Positionen des C-Atoms, an das die Aminogruppe gebunden ist, unsubstituiert ist, dadurch gekennzeichnet, daß
a) Dimethylsuccinylsuccinat mit einem oder mehreren Alkoholen der Formel
R₃-OH II
in der R₃ eine unsubstituierte oder durch Phenyl substituierte Alkylgruppe mit 3 bis 22 C-Atomen bedeutet in Gegenwart eines sauren Katalysators unter Druck bei Temperaturen von 60 bis 180 °C erhitzt wird, wobei eine Verbindung der Formel 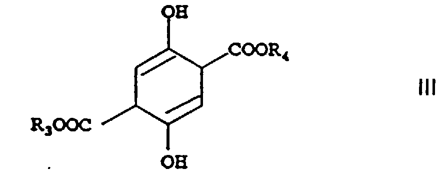 in der R₃ die obgenannte Bedeutung hat und R₄ die Bedeutung von R₃ hat und zusätzlich eine Methylgruppe bedeutet, wobei R₃ und R₄ in der Formel III austauschbar sind, erhalten wird,
b) das Reaktionsprodukt aus Stufe a) nach oder ohne Isolierung gegebenenfalls unter Zugabe eines unter den Reaktionsbedingungen inerten Verdünnungsmittels mit mindestens der zweifachen molaren Menge eines oder mehrerer Amine der Formel
R₁-NH₂ und/oder R₂-NH₂ IV
in der R₁ und R₂ die obgenannte Bedeutung haben in Gegenwart von Säure auf Temperaturen von 40 bis 150 °C erhitzt wird wobei eine Verbindung der Formel 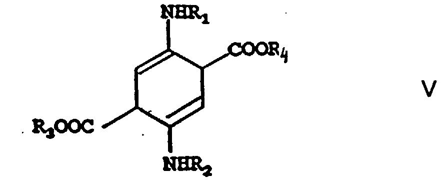 in der R₁, R₂, R₃ und R₄ die obgenannten Bedeutungen haben, erhalten wird,
c) das Reaktionsprodukt aus Stufe b) nach oder ohne Isolierung gegebenenfalls unter Zugabe eines unter den Reaktionsbedingungen inerten Verdünnungsmittels mit Säure bis zur sauren Reaktion versetzt und auf Temperaturen von 50 bis 100 °C erhitzt wird, worauf Luft in das Reaktionsgemisch eingeblasen wird, wobei der Terephthalsäureester der Formel 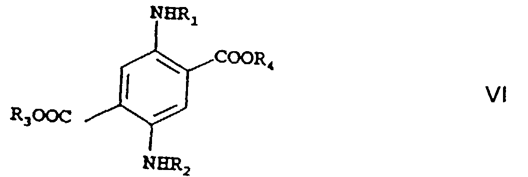 in der R₁, R₂, R₃ und R₄ die obgenannte Bedeutung haben, erhalten wird, der
d) nach oder ohne Isolierung gegebenenfalls unter Zugabe eines unter den Reaktionsbedingungen inerten Verdünnungsmittels durch Umsetzung mit einer Verbindung der Formel M-OH, in der M ein Metall bedeutet, unter Erhitzen in eine Verbindung der Formel 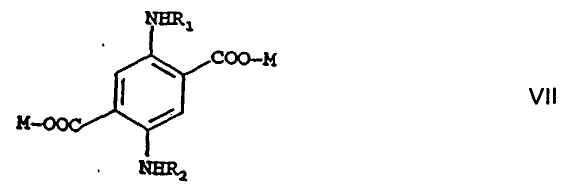 übergeführt wird, die
e) nach oder ohne Isolierung gegebenenfalls unter Zugabe eines unter den Reaktionsbedingungen inerten Verdünnungsmittels mit Wasser und Säure bis zur sauren Reaktion versetzt und zur Terephthalsäure der Formel I reagieren gelassen wird, die aus dem Reaktionsgemisch isoliert wird.

7. Verfahren nach Anspruch 6, dadurch gekennzeichnet, daß ein Amin der Formel IV, in der R₁ und R₂ gleich sind und eine unsubstituierte oder durch Halogenatome, Alkyl- oder Alkoxygruppen mit jeweils 1 bis 4 C-Atomen oder Nitrogruppen substituierte Phenylgruppe bedeuten, eingesetzt wird.

8. Verfahren nach einem der Ansprüche 6 oder 7, dadurch gekennzeichnet, daß das Verfahren in einem Alkohol der Formel II nach Anspruch 6 als Verdünnungsmittel durchgeführt wird.

9. Verfahren nach einem der Ansprüche 6 bis 8, dadurch gekennzeichnet, daß das Verfahren als Eintopfreaktion durchgeführt wird.

## Claims

1. Process for the transesterification of dimethyl succinylsuccinate, characterized in that dimethyl succinylsuccinate is reacted with one or more aliphatic, straight-chain, branched or cyclic monoalcohols which are unsubstituted or substituted by phenyl groups and in each case have 2 to 22 C atoms, in the presence of an acidic catalyst and in the presence or absence of a diluent which is inert under the reaction conditions with exclusion of oxygen and under pressure, at least one of the two methoxy groups being replaced by the oxy group corresponding to the alcohol used.

2. Process according to Claim 1, characterized in that the alcohol employed is an unsubstituted, aliphatic, straight-chain or branched monoalcohol having 2 to 16 C atoms.

3. Process according to Claim 2, characterized in that the alcohol employed is an unsubstituted, aliphatic, straight-chain or branched monoalcohol having 3 to 8 C atoms.

4. Process according to one of Claims 1 to 3, characterized in that the alcohol is simultaneously employed as a diluent.

5. Process according to one of Claims 1 to 4, characterized in that the acidic catalyst employed is a mineral acid or a strongly acidic cation exchanger.

6. Process for the preparation of 2,5-dianilinoterephthalic acids of the formula 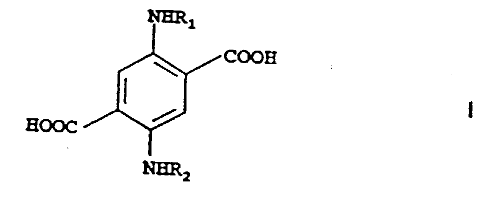 in which R₁ and R₂ are identical or different and in each case are a phenyl or naphthyl group which is unsubstituted or mono- or polysubstituted by halogen, nitro, alkyl, alkoxy, phenyl, phenoxy or trifluoromethyl or by alkyl-substituted carbamoyl groups, at least one of the alpha positions of the C atom to which the amino group is bonded being unsubstituted, characterized in that
a) dimethyl succinylsuccinate is heated with one or more alcohols of the formula
R₃-OH II
in which R₃ is an alkyl group which is unsubstituted or substituted by phenyl and has 3 to 22 C atoms, in the presence of an acidic catalyst under pressure at temperatures from 60 to 180°C, a compound of the formula 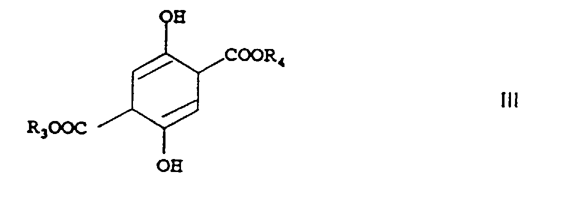 in which R₃ has the abovementioned meaning and R₄ has the meaning of R₃ and is additionally a methyl group, R₃ and R₄ in the formula III being exchangeable, being obtained,
b) the reaction product from stage a) is heated to temperatures of 40 to 150°C after or without isolation, optionally with addition of a diluent which is inert under the reaction conditions, with at least a two-fold molar amount of one or more amines of the formula
R₁-NH₂ and/or R₂-NH₂ IV
in which R₁ and R₂ have the abovementioned meaning, in the presence of acid, a compound of the formula 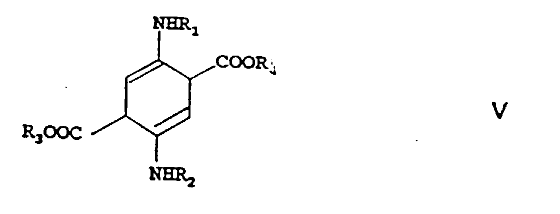 in which R₁, R₂, R₃ and R₄ have the abovementioned meanings, being obtained,
c) the reaction product from stage b) is treated with acid until the reaction is acidic and heated to temperatures of 50 to 100°C after or without isolation, optionally with addition of a diluent which is inert under the reaction conditions, after which air is blown into the reaction mixture, the terephthalate ester of the formula 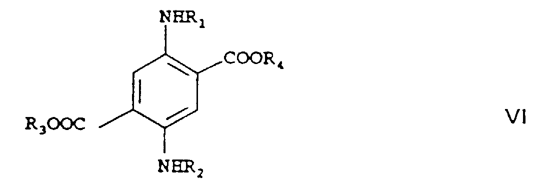 in which R₁, R₂, R₃ and R₄ have the abovementioned meaning, being obtained, which
d) is converted with heating after or without isolation, optionally with addition of a diluent which is inert under the reaction conditions, by reaction with a compound of the formula M-OH, in which M is a metal, to a compound of the formula 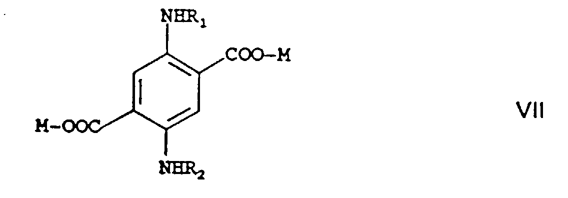 which
e) is treated with water and acid until the reaction is acidic and allowed to react after or without isolation, optionally with addition of a diluent which is inert under the reaction conditions to give the terephthalic acid of the formula I which is isolated from the reaction mixture.

7. Process according to Claim 6, characterized in that an amine of the formula IV is employed in which R₁ and R₂ are identical and are a phenyl group which is unsubstituted or substituted by halogen atoms, alkyl or alkoxy groups in each case having 1 to 4 C atoms or nitro groups.

8. Process according to one of Claims 6 or 7, characterized in that the process is carried out in an alcohol of the formula II according to Claim 6 as a diluent.

9. Process according to one of Claims 6 to 8, characterized in that the process is carried out as a one-pot reaction.

## Revendications

1. Procédé de transestérification de succinate de diméthylsuccinyle, caractérisé en ce qu'on fait réagir un succinate de diméthylsuccinyle avec un ou plusieurs monools aliphatiques, linéaires, ramifiés ou cycliques, contenant chacun de 2 à 22 atomes de carbone, non substitués ou substitués par des groupes phényle, en présence d'un catalyseur acide, en présence ou en l'absence d'un diluant inerte dans les conditions de réaction, à l'abri de l'oxygène et sous pression, au moins un des deux groupes méthoxy étant remplacé par le groupe oxy correspondant à l'alcool utilisé.

2. Procédé selon la revendication 1, caractérisé en ce qu'on utilise, comme alcool, un monool aliphatique non substitué, linéaire ou ramifié, contenant de 2 à 16 atomes de carbone.

3. Procédé selon la revendication 2, caractérisé en ce qu'on utilise, comme alcool, un monool aliphatique non substitué, linéaire ou ramifié, contenant de 3 à 8 atomes de carbone.

4. Procédé selon l'une des revendications 1 à 3, caractérisé en ce que l'alcool sert en même temps de diluant.

5. Procédé selon l'une des revendications 1 à 4, caractérisé en ce qu'on utilise, comme catalyseur acide, un acide minéral ou un échangeur de cations fortement acide.

6. Procédé de préparation d'acides 2,5-dianilinotéréphtaliques répondant à la formule 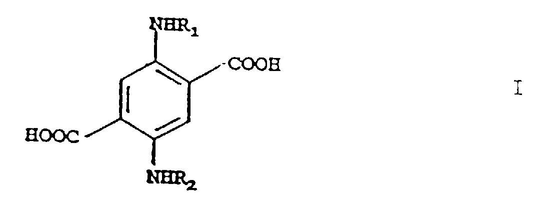 où R₁ et R₂ sont identiques ou différents et signifient chacun un groupe phényle ou naphtyle non substitué ou mono- ou polysubstitué par un halogène ou par un groupe nitro, alkyle, alcoxy, phényle, phénoxy ou trifluorométhyle ou substitué par des groupes carbamoyles eux-mêmes substitués par un groupe alkyle, au moins une des positions alpha de l'atome de carbone auquel le groupe amino est lié étant non substituée, caractérisé en ce que
a) on chauffe du succinate de diméthylsuccinyle avec un ou plusieurs alcools répondant à la formule
R₃-OH II
où R₃ signifie un groupe alkyle ayant de 3 à 22 atomes de carbone, non substitué ou substitué par le groupe phényle, en présence d'un catalyseur acide, sous pression, à des températures de 60 à 180°C, et on obtient un composé répondant à la formule 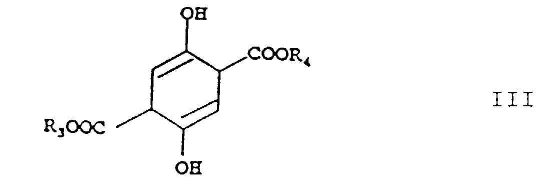 où R₃ a la signification indiquée ci-dessus, et R₄ a la signification de R₃ et signifie, en plus, le groupe méthyle, R₃ et R₄ étant interchangeables dans la formule III, b) après isolement ou sans isolement, éventuellement avec addition d'un diluant inerte dans les conditions de réaction, on chauffe le produit de réaction obtenu dans l'étape a) avec une quantité molaire au moins double d'une ou plusieurs amines répondant à la formule
R₁-NH₂ et/ou R₂-NH₂ IV
où R₁ et R₂ ont les significations indiquées ci-dessus, en présence d'un acide, à des températures de 40 à 150°C, et on obtient un composé répondant à la formule 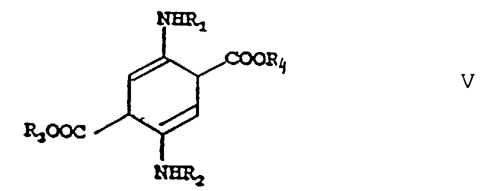 où R₁, R₂, R₃ et R₄ ont les significations indiquées ci-dessus,
c) après un isolement ou sans isolement, éventuellement avec addition d'un diluant inerte dans les conditions de réaction, on mélange le produit de réaction obtenu dans l'étape b) avec un acide jusqu'à l'obtention d'une réaction acide et on chauffe le mélange résultant à des températures de 50 à 100°C, après quoi on fait barboter de l'air dans le mélange de réaction et on obtient l'ester téréphtalique répondant à la formule 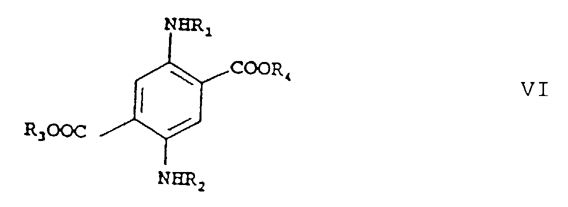 où R₁, R₂, R₃ et R₄ ont les significations indiquées ci-dessus,
d) qu'on transforme, après un isolement ou sans isolement. éventuellement avec addition d'un diluant inerte dans les conditions de réaction, par réaction avec un composé de formule M-OH, où M signifie un métal, avec chauffage, en un composé répondant à la formule 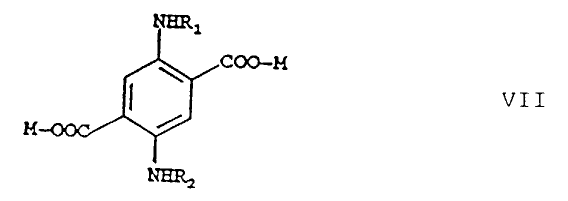
e) auquel on ajoute de l'eau et un acide jusqu'à l'obtention d'une réaction acide, après un isolement ou sans isolement, éventuellement avec addition d'un diluant inerte dans les conditions de réaction, et on le laisse réagir pour donner l'acide téréphtalique de formule I qu'on isole du mélange de réaction.

7. Procédé selon la revendication 6, caractérisé en ce qu'on utilise une amine de formule IV où R₁ et R₂ sont identiques et signifient un groupe phényle non substitué ou substitué par des atomes d'halogène, par des groupes alkyles ou alcoxy ayant chacun de 1 à 4 atomes de carbone ou par des groupes nitro.

8. Procédé selon la revendication 6 ou 7, caractérisé en ce qu'on le met en oeuvre-dans un alcool de formule II selon la revendication 6 comme diluant.

9. Procédé selon l'une des revendications 6 à 8, caractérisé en ce qu'on le met en oeuvre sous la forme d'une réaction dans un récipient unique.
